# EUROPEAN PATENT SPECIFICATION

(11) **EP 1 663 191 B1**
(45) Date of publication and mention of the grant of the patent: **20.01.2010**
(21) Application number: 04741403.2
(22) Date of filing: 09.08.2004
(51) Int. Cl.: A61K 31/155, A61K 31/4439, A61K 9/28, A61P 3/10

(54) **COMPOSITION COMPRISING ROSIGLITAZONE AND METFORMIN**
ZUBEREITUNG MIT ROSIGLITAZON UND METFORMIN
COMPOSITION COMPRENANT DE LA ROSIGLITAZONE ET DE LA METFORMINE

(30) Priority: 11.08.2003 GB 0318824
(43) Date of publication of application: 07.06.2006
(73) Proprietor: SmithKline Beecham (Cork) Limited, Carrigaline Cork (IE)
(72) Inventor: COLES, Peter John, GlaxoSmithKline Inc., Mississauga, Ontario L5N 6L4 (CA); MACKENZIE, Donald Colin, GlaxoSmithKline (CIDRA), King of Prussia Pennsylvania 19406 (US); MUDD, Paul Norman, Jr., GlaxoSmithKline, Research Triangle Park, NC 27709 (US)
(74) Representative: Rutter, Keith
(86) International application number: PCT/EP2004/008970
(87) International publication number: WO 2005/013956

(56) References cited:
- EP-A- 0 365 123
- WO-A-01/35940
- WO-A-01/82867
- WO-A-03/068195
- US-A- 6 099 862

## Description

The present invention relates to an oral dosage form comprising 5-[4-[2-(N-methyl-N-(2 pyridyl)amino)ethoxy]benzyl]thiazolidine-2,4-dione (hereinafter 'Compound A') or a pharmaceutically acceptable salt or solvate thereof and another antidiabetic agent, to a process for preparing such a dosage form and to the use of such a dosage form in medicine.

The use of a coating to control the rate of release of an active agent has received considerable attention and many different devices have been developed for such a purpose. For example, International Patent Application, Publication Number WO 01/05430 describes a drug delivery device that enables the delivery of drug substances which exhibit pH dependent solubility, in particular compounds that are more soluble at low pH levels (less than pH 2) than at near neutral levels (greater than about pH 5). Such delivery devices are characterised by the presence of a coating that is impermeable and insoluble in the fluid of the environment of use.

International patent application, Publication Number WO 95/30422 describes a series of controlled-release dosage forms of azithromycin. In particular, there is described a series of dosage forms that reduce the exposure of the upper GI tract (e.g. the stomach) to high concentrations of azithromycin, by the use of a pH dependent coating. Such dosage forms do not feature openings through which release of the drug substance may occur.

US Patent Number 6,099,859 describes a controlled release tablet for the delivery of an antihyperglycaemic drug, which comprises an osmotically active drug-containing core and a semipermeable membrane, wherein the semipermeable membrane is permeable to the passage of water and biological fluids and is impermeable to the passage of the drug substance. The semipermeable membrane contains at least one passageway for the release of the antihyperglycaemic drug.

US Patent Number 5,543,155 describes a diffusion-osmotic controlled drug release pharmaceutical composition comprising a one- or two-layer tablet core containing hydroxypropyl methylcellulose, said core having a film-coat comprising an ammonium methacrylate copolymer.

Additional devices that utilise a coating to control the rate of release of an active agent are discussed in US Patent Number 5,004,614. This patent describes a tablet core provided with an outer coating that is substantially impermeable to environmental fluid. The said outer coating may be prepared from materials that are either insoluble or soluble in the environmental fluids. Where a soluble material is used, the coating is of sufficient thickness that the core is not exposed to environmental fluid before the desired duration of the controlled release of the active agent has passed. Through this impermeable outer coating, one or more opening(s) has been created, so as to provide environmental fluids with an access route to the core. Therefore, upon ingestion of the coated tablet, gastro-intestinal fluid can enter the opening(s) and contact or penetrate the core, to release the active agent. The result is that the active agent is released in a controlled manner out of the opening(s) only. The preferred geometry is such that there is a circular hole on the top and bottom face of the coated tablet. The opening(s) in question have an area from about 10 to 60 percent of the face area of the coated tablet. The rate of drug release is found to be directly related to the diameter of the opening(s) and to the solubility of the matrix core and active agent, allowing the possibility for a variety of drug release profiles be it zero or first order release.

The substantially impermeable coatings of US 5,004,614 are not suitable for the controlled release of all active agents, especially pharmaceutically active weak bases or pharmaceutically acceptable salts and solvates thereof. Such active agents exhibit a marked pH dependent solubility, *i.e*. they are more soluble at around pH 2, associated with regions found in the stomach, compared to their solubility in the generally neutral conditions of the small intestine, around pH 7.

International Patent Application, Publication Number WO 03/068195 discloses an oral dosage form comprising an erodable core which contains a pharmaceutically active weak base or a pharmaceutically acceptable salt or solvate thereof, such as Compound A, the core having a coating with one or more openings leading to the core, and the coating being erodable under predetermined pH conditions. This provides a beneficial means for administration of a pharmaceutically active weak base or a pharmaceutically acceptable salt or solvate thereof, such as Compound A, where it is desirable that release of the active compound takes place in more than one pH environment, based on the finding that it is also beneficial for the coating to be erodable or soluble in a pH dependent manner.

We have now found that the oral dosage form described in International Patent Application Number WO 03/068195 may be beneficially used as a platform for the delivery of more than one active agent, such as, for example, Compound A or a pharmaceutically acceptable salt or solvate thereof and metformin or a pharmaceutically acceptable salt or solvate therof. To this end the said oral dosage form provides a beneficial means for delivering metformin, which has a narrow absorption window.

European Patent Application, Publication Number 0 306 228 A1 relates to certain thiazolidinedione derivatives disclosed as having antihyperglycaemic and hypolipidaemic activity. One particular thiazolidinedione disclosed in EP 0 306 228 A1 is Compound A. International Patent Application, Publication Number WO 94/05659 discloses certain salts of Compound A including the maleate salt at Example 1 thereof. Compound A or a pharmaceutically acceptable salt thereof or a pharmaceutically acceptable solvate thereof, may be prepared using known methods, for example those disclosed in EP 0 306 228 and WO 94/05659.

Compound A is a pharmaceutically acceptable weak base.

Compound A and pharmaceutically acceptable salts or solvates thereof have useful pharmaceutical properties. In particular, Compound A or a salt or solvate thereof is indicated to be useful for the treatment and/or prophylaxis of diabetes mellitus, conditions associated with diabetes mellitus and certain complications thereof; metabolic syndrome, impaired glucose tolerance and impaired fasting glucose.

International Patent Application, Publication Number WO 01/35941 describes certain fixed dose compositions comprising a thiazolidinedione, such as Compound A or a pharmaceutically acceptable derivative thereof and another antidiabetic agent such as metformin hydrochloride.

European Patent Number 0 861 666 describes pharmaceutical compositions comprising insulin sensitisers, such as pioglitazone or Compound A, and metformin.

International Patent Application, Publication Number WO 00/28989 describes various modified release pharmaceutical compositions comprising Compound A or a pharmaceutically acceptable salt or solvate thereof, and another antidiabetic agent.

US Patent Application, Publication Number US 2003/0187074 describes an oral delivery system comprising a biguanide, such as metformin hydrochloride, which provides controlled release of the biguanide independent of environmental pH.

US Patent Numbers 6,475,521 and 6,660,300 describe controlled release delivery systems for pharmaceuticals having high water solubility, such as metformin hydrochloride.

Compound A and pharmaceutically acceptable salts or solvates thereof, in particular the maleate salt, are known to exhibit marked pH dependent solubility, i.e. they are more soluble in the acidic conditions of the stomach (around pH 2) than in the near neutral conditions of the lower intestine (around pH 7).

Certain antidiabetic agents, such as metformin, are known to have a narrow absorption window. It is therefore preferable that such agents are delivered substantially exclusively in a particular pharmacological environment, such as the stomach.

Thus, it is an object of the present invention to provide an oral dosage which compensates for the pH dependent solubility of Compound A or a pharmaceutically acceptable salt or solvate thereof, and which compensates for the narrow absorption window of metformin, by providing delivery of the metformin substantially exclusively in a particular pharmacological environment, such as the stomach. Such a dosage form is indicated to provide a beneficial effect on glycemic control for an extended period of time. Such a dosage form is also considered to be suitable for once daily administration.

Accordingly, in its broadest aspect the present invention provides an oral dosage form comprising an erodable core, which core comprises Compound A or a pharmaceutically acceptable salt or solvate thereof and metformin or a pharmaceutical acceptable salt thereof, the core having a coating with one or more openings leading to the core characterised in that the coating is erodable under predetermined pH conditions.

The present invention further provides an oral dosage form comprising,
(i) an erodable core, which core comprises Compound A or a pharmaceutically acceptable salt or solvate thereof and metformin or a pharmaceutically acceptable salt or solvate thereof; and
(ii) an erodable coating around said core, which coating comprises one or more openings extending substantially completely through said coating but not substantially penetrating said core and communicating from the environment of use to said core; wherein release of Compound A or a pharmaceutically acceptable salt or solvate thereof and the metformin or a pharmaceutically acceptable salt or solvate thereof from the erodable core occurs substantially through the said opening(s) and through erosion of said erodable coating under pre-determined pH conditions.

Suitably, the dosage form is a tablet.

The above references to the core being erodable includes the situation where the core disintegrates partially or wholly, or dissolves, or becomes porous, on contact with an environmental fluid so as to allow the fluid to contact the active agent. Suitably, the core disintegrates partially. Suitably, the core disintegrates wholly. Suitably, the core dissolves. Suitably, the core becomes porous.

While this invention provides that erosion of the coating is pH-dependent, the core may release Compound A or a pharmaceutically acceptable salt or solvate thereof and the metformin or a pharmaceutically acceptable salt or solvate thereof by eroding in a non-pH dependent manner. However, to suit a specific demand, the core may be a material which allows pH dependent erosion or disintegration of the core to release Compound A or a pharmaceutically acceptable salt or solvate thereof and the other metformin or a pharmaceutically acceptable salt or solvate thereof from its matrix.

In one embodiment, the core is formulated so as to be erodable to substantially the same extent in both the stomach and the intestines.

The erodable core may be formulated to provide immediate or modified release of at least one of Compound A or a pharmaceutically acceptable salt or solvate thereof and metformin or a pharmaceutically acceptable salt or solvate thereof. Suitably, the core is formulated to provide immediate release of both Compound A or a pharmaceutically acceptable salt or solvate thereof and the metformin or a pharmaceutically acceptable salt or solvate thereof. In the alternative, the core is formulated to provide modified release of both Compound A or a pharmaceutically acceptable salt or solvate thereof and the metformin or a pharmaceutically acceptable salt or solvate thereof.

Suitable materials for the core include erodable polymethylmethacrylate resins such as the Eudragit™ series, for example Eudragit™ L30D, saccharoses, for example lactose and maltose, and cellulose esters, for example methylcellulose, hydroxypropylmethylcellulose (HPMC) and hydroxypropylcellulose, magnesium stearate, sodium starch glycolate and povidone (polyvinylpyrrolidone). Suitably, the core is predominantly microcrystalline cellulose, hydroxypropylmethylcellulose, lactose and povidone. More suitably, the core consists essentially of hydroxypropylmethylcellulose, lactose, microcrystalline cellulose, sodium starch glycolate, povidone and magnesium stearate.

The above reference to the coating being erodable includes the situation where the coating disintegrates partially or wholly, or dissolves, or becomes porous, on contact with an environmental fluid so as to allow the fluid to contact the core. Suitably, the coating disintegrates partially. Suitably, the coating disintegrates wholly. Suitably, the coating dissolves. Suitably, the coating becomes porous. Preferably, the erodable coating is an enteric coating, *i.e.* it has a defined, pre-determined pH threshold at which it dissolves. Preferably, the coating erodes at pH greater than 4.5. More preferably, the coating erodes in the pH range from 4.5 to 8. Most preferably, the coating erodes in the pH range 5 to 7. Preferably, the enteric coating is non-permeable.

The use of a coating that erodes rapidly on exiting the stomach environment has been found to be particularly beneficial given that metformin, has a narrow absorption window. In such circumstances any active agent that is not released in the stomach is rapidly delivered on entry into the small intestine, thereby minimising any loss in absorption associated with delivery lower down the GI tract.

Materials and their blends suitable for use as a pH-dependent erodable coating material in this invention include various polymethacrylate polymers, coprocessed polyvinylacetate phthalate, cellulose acetate trimellitate, cellulose acetate phthalate, shellac, hydroxyropylmethylcellulose phthalate polymers and their copolymers. Suitably, the coating material is selected from cellulose acetate trimellitate (CAT), polyvinyl acetate phthalate, hydroxypropylmethylcellulose phthalate 50, hydroxpropylnethylcellulose phthalate 55, Acryl-eze™, Aquateric™, cellulose acetate phthalate, Eudragit™ L30 D, Eudragit™ L, Eudragit™ S and shellac. Most preferably, the coating material is Eudragit™ L30 D.

When necessary, the erodable coating may be modified by addition of plasticisers or anti-tack agents. Suitable materials for this purpose include waxy materials such as glycerides, for example glyceryl monostearate.

Typical sizes for the opening(s), when circular, to be formed in the coating are in the range 0.5 mm - 8 mm of diameter, such as 1, 2, 3, 4, 5 or 6 mms in diameter, depending on the overall size of the tablet and the desired rate of release. The opening(s) may have any convenient geometrical shape, but a rounded shape, e.g. substantially circular or elliptical, is generally preferred. More elaborate shapes, such as text characters or graphics, may also be formed, provided that the release rate can be made uniform in individual dosage forms. Typical sizes of non-circular openings are equivalent in area to the above mentioned sizes for circular openings, thus in the range of from about 0.19 to about 50.3 mm².

For the purposes of the present invention, the term "opening" is synonymous with hole, aperture, orifice, passageway, outlet etc.

The opening(s) may be formed by methods disclosed in US 5,004,614. Typically opening(s) may be formed by drilling, for example using mechanical drill bits or laser beams, or by punches that remove the cut area. The formation of the opening(s) may by default remove a small portion of the exposed core. It is also possible to purposely form a cavity below the aperture as a release rate controlling device, the cavity exposing a greater initial surface area of core than a flat surface. Suitably, the opening(s) extend through the entire erodable coating such that there is immediate exposure of the core to the environmental fluid when the device is placed in the desired environment of use.

Also it is possible to form the opening(s) *in situ* when the dosage form is administered, by forming a coating containing pore-forming agents i.e. material that will dissolve in the stomach to create pores in the coating. Accordingly, there is also provided an oral dosage form comprising,
(i) an erodable core, which core comprises Compound A or a pharmaceutically acceptable salt or solvate thereof and metformin or a pharmaceutically acceptable salt or solvate thereof; and
(ii) an erodable coating surrounding said core, which coating comprises a pore forming agent that is erodable in the pH range from 1 to 3 to form one or more openings extending substantially completely through said coating but not substantially penetrating said core and communicating from the environment of use to said core;
wherein release of Compound A or a pharmaceutically acceptable salt or solvate thereof and the metformin or a pharmaceutically acceptable salt or solvate thereoffrom the dosage form occurs through the said opening(s) by the erosion of said erodable core and through erosion of said erodable coating under pre-determined pH conditions.

In US 5,004,614, the opening(s) preferably comprise about 10 - 60 % of the total face area of the tablet i.e. the upper and lower surfaces of a biconvex tablet. In the present invention, the opening(s) may comprise 0.25 to 70%, such as 10 - 70% of the total face area.

Alternatively, it may be useful to characterise the rate controlling effect of the opening(s) by reference to the area of the opening(s) relative to the total surface area of the coated tablet. Additionally, especially in cases where the core erodes by undercutting of the edges of the opening(s), the rate controlling effect may be related to the total circumference of the opening(s).

An unexpected finding is that two openings, for example one on each primary surface of a biconvex tablet, release an active agent from the core at a rate marginally greater than that of a single opening of the same overall area. It is also indicated that the variability of the release rate from the two openings is less than the variability of release rate from the corresponding single opening. Accordingly, in one embodiment of the invention, the coating of the core is provided with two or more openings. More preferably, the erodable coating surrounding the core is provided with two openings extending substantially completely through said coating but not substantially penetrating said core and communicating from the environment of use to said core.

Where more than one opening is provided, the openings may be located on the same face of the oral dosage form, or on different surfaces. Suitably, the oral dosage form has two openings, one on each opposing surface. Suitably, the oral dosage form is a tablet having two opposed primary surfaces, each surface having one opening through the coating.

As a protection for the core material, to prevent contamination via the opening(s) before dosing, it may desirable to provide a conventional seal coating to either the core, or to the dosage form after formation of the opening(s). The seal coat may be a sub-coat or over-coat to the erodable coating.

Where the oral dosage form comprises an antidiabetic agent which is known to have a narrow absorption window, such as metformin, the dosage form is preferably formulated to provide delivery of the metformin or a pharmaceutically acceptable salt or solvate thereof substantially exclusively in a particular pharmacological environment, such as the stomach. Where substantially exclusive delivery of the other antidiabetic agent in the stomach is required, the oral dosage form is suitably formulated to reside in the gastric environment over an extended period of time. Increased gastric retention times may be achieved, for example, by increasing the size of the dosage form, and/or administering the dosage form with food.

According to a further aspect of the present invention, there is provided a process for the preparation of an oral dosage form according to the present invention, which process comprises:
(a) preparing an erodable tablet core comprising Compound A or a pharmaceutically acceptable salt or solvate thereof and another antidiabetic agent;
(b) coating the core with a material with pH-dependent erodability; and
(c) creating one or more openings in the coating.

According to yet a further aspect of the present invention there is provided a process for the preparation of an oral dosage form according to the present invention, which process comprises:
(a) preparing an erodable tablet core comprising Compound A or a pharmaceutically acceptable salt or solvate thereof and another antidiabetic agent;
(b) coating the core with a material with pH-dependent erodability; and
(c) creating one or more openings in the coating, said opening(s) extending substantially completely through said coating but not substantially penetrating said core and communicating from the environment of use to said core.

The core may be prepared by compressing suitable ingredients to form a compacted mass, which comprises the core of the dosage form (also referred to herein as "tablet core"). This may be prepared using conventional tablet excipients and formulation compression methods. Thus, the core typically comprises the active agents along with excipients that impart satisfactory processing and compression characteristics such as diluents, binders and lubricants. Additional excipents that may form part of the core of the device include disintegrants, flavourants, colorants, release modifying agents and/or solubilising agents such as surfactants, pH modifiers and complexation vehicles. Typically the active agents and excipients are thoroughly mixed prior to compression into a solid core. The core of the device may be formed by wet granulation methods, dry granulation methods or by direct compression. The core may be produced according to any desired pre-selected shape such as bi-convex, hemi-spherical, near hemi-spherical, round, oval, generally ellipsoidal, oblong, generally cylindrical or polyhedral, e.g. a triangular prism shape. The term "near hemi-spherical" is intended to be construed in the manner described in US 5,004,614. Suitably the core is formulated into a bi-convex shape, e.g. having two domed opposite surfaces. In addition, the core may be produced in a multi-layered (e.g. bi- or tri- layered) form. For example, the core may be formulated as a bilayer, in which one layer comprises Compound A or a pharmaceutically acceptable salt or solvate thereof and the other layer comprises another antidiabetic agent.

The core may be coated with a suitable pH dependent erodable material by any pharmaceutically acceptable coating method. Examples include coating methods disclosed in US 5,004,614 and film coating, sugar coating, spray coating, dip coating, compression coating, electrostatic coating. Typical methods include spraying the coating onto the tablet core in a rotating pan coater or in a fluidised bed coater until the desired coating thickness is achieved. Suitably the coating is provided to add about 4 to 8 mg/ cm² or 5 - 7 mg/ cm² of dry polymer around the tablet surface area. Typically this results in an increase in weight (relative to the core) of from 3 -10% or 5 - 10 % by weight. Suitably, the coating has a thickness in the range 0.05 to 0.5 mm.

As used herein, the term "modified release" means a composition which has been designed to produce a desired pharmacokinetic profile by choice of formulation. Modified release also includes modified release compositions in combination with non-modified release compositions. For example, the term "modified release" shall comprise delayed, pulsed and sustained release either alone or in any combination.

In one aspect the modified release composition provides delayed release of at least one of Compound A or a pharmaceutically acceptable salt or solvate thereof and metformin or a Pharmaceutical acceptable salt or solvate thereof. Delayed release is conveniently obtained by use of a gastric resistant formulation such as an enteric formulation. Such an enteric formulation may comprise multi-particulates, such as multi-particulate spheres, coated with a gastric resistant polymer. Suitable, gastric resistant polymers include polymers derived from methacrylates, cellulose acetate phthalate, polyvinyl acetate phthalate and hydroxypropyl methylcellulose phtahlate. Examples of such polymers include Eudragit L100-55™ (Poly(methacrylic acid, ethyl acrylate) 1:1) for example as Eudragit L30D-55™ or Eudragit FS 30D™, Aquateric™ (cellulose acetate phthalate), Sureteric™ (polyvinyl acetate phthalate), HPMCP-HP-55S™ (hydroxypropyl methylcellulose phtahlate).

The multiparticulates include coated drug-coated non-pareil substrates, such as lactose spheres, or drug containing non-pareil substrates, such as drug containing lactose spheres. Such multiparticulates are coated as required with an appropriate enteric formulation, for example a polymethacrylate polymer. An example of a suitable polymethacrylate polymer is Eudragit L100-55™ (Poly(methacrylic acid, ethyl acrylate) 1:1), for example as Eudragit L30D-55™ or Eudragit FS 30D™.

In a further aspect the modified release composition provides sustained release of at least one of Compound A or a pharmaceutically acceptable salt or solvate thereof and metformin or a pharmaceutically acceptable salt or solvate thereof, for example providing release of the active agent(s) over a time period of up to 26 hours; suitably in the range of 4 to 24 hours; preferably in the range of 12 to 24 hours.

Sustained release is typically provided by use of a sustained release matrix, usually in tablet form, such as disintegrating, non-disintegrating or eroding matrices.

Sustained release is suitably obtained by use of a non-disintegrating matrix tablet formulation. Suitable non disintegrating matrix tablet formulations are provided by the incorporation of methacrylates, cellulose acetates, carbomers and hydroxypropyl methylcellulose phtahlate into the tablet. Examples of suitable materials include Eudragit RS™ (Poly(ethyl acrylate, methyl methacrylate, trimethylammonioethyl methacrylate chloride) 1:2:0.1), Eudragit RL™ (Poly(ethyl acrylate, methyl methacrylate, trimethylammonioethyl methacrylate chloride) 1:2:0.2), Carbopol 971 P™ (carbomer), HPMCP-HP-55S™ (hydroxypropyl methylcellulose phtahlate).

Sustained release is further obtained by use of a disintegrating matrix tablet formulation, for example by incorporating methacrylates, methylcellulose or hydroxypropyl methylcellulose into the tablet. Examples of suitable materials include Eudragit L™ (Poly(methacrylic acid, ethyl acrylate) 1:1) and Methocel K4M™ (hydroxypropyl methylcellulose).

Sustained release can also be achieved by using multiparticulates coated with semipermeable membranes. The multiparticulates include coated drug-coated non-pareil substrates, such as lactose spheres, or drug containing substrates, such as drug containing lactose/Avicel™ (microcrystalline cellulose) spheres. Such multiparticulates are coated as required with the appropriate semi-permeable membranes, such as ethylcellulose polymer.

In yet a further aspect the modified release composition provides pulsed release of at least one of Compound A or a pharmaceutically acceptable salt or solvate thereof and metformin or a pharmaceutically acceptable salt or solvate thereof, for example providing up to 4, for example 2, pulses of active agent per 24 hours.

Suitable materials for an immediate release composition include saccharoses, for example lactose and maltose, and cellulose, for example microcrystalline cellulose. Most suitably, the immediate release composition is predominantly microcrystalline cellulose. More suitably, the immediate release composition consists essentially of lactose, microcrystalline cellulose and magnesium stearate.

As indicated above, the oral dosage form of the present invention is considered to be suitable for once daily administration and during use is indicated to provide a therapeutic effect over an extended period of time, such as up to 24 hours, for example, up to 12, 14, 16, 18, 20 and 24 hours, per unit dose.

A suitable dosage for of Compound A or a pharmaceutically acceptable salt or solvate thereof when used in accordance with the present invention is up to 12 mg, for example, 1 to 12 mg. Thus, suitable dosage forms comprise 1, 2, 3, 4, 5, 6, 7, 8, 9, 10, 11 or 12 mg of Compound A or a pharmaceutically acceptable salt or solvate thereof.

Particular dosage forms comprise 2 to 4 mg of Compound A or a pharmaceutically acceptable salt or solvate thereof.

Particular dosage forms comprise 4 to 8 mg of Compound A or a pharmaceutically acceptable salt or solvate thereof.

Particular dosage forms comprise 8 to 12 mg of Compound A or a pharmaceutically acceptable salt or solvate thereof.

One dosage form comprises 2 mg of Compound A or a pharmaceutically acceptable salt or solvate thereof.

Preferred dosage forms comprise 4 mg of Compound A or a pharmaceutically acceptable salt or solvate thereof.

Preferred dosage forms comprise 8 mg of Compound A or a pharmaceutically acceptable salt or solvate thereof. A preferred pharmaceutically acceptable salt of metformin is the hydrochloride salt.

Suitable dosages, preferably unit dosages, of the metformin or a pharmaceutically acceptable salt or solvate thereof, include the known permissible doses for this compound as described or referred to in reference texts such as the British and US Pharmacopoeias, Remington's Pharmaceutical Sciences (Mack Publishing Co.), Martindale The Extra Pharmacopoeia (London, The Pharmaceutical Press) (for example see the 31 st Edition page 341 and pages cited therein) or the above mentioned publications.

A suitable dosage of metformin is between 100 to 3000 mg, for example 250, 500 mg, 850 mg or 1000 mg, especially 500 mg and 1000 mg.

Where the dosage form comprises Compound A or a pharmaceutically acceptable salt or solvate thereof and metformin, particularly preferred fixed doses are 2 mg Compound A and 500 mg metformin; 4 mg Compound A and 500 mg metformin; 2 mg Compound A and 1000 mg metformin; 4 mg Compound A and 1000 mg metformin; and 8 mg Compound A and 1000 mg metformin.

By adjustment of the above variables and the surface area of the exposed core, the release rates in the different environmental conditions can be harmonised to obtain comparable release rates under different body environments, and so achieve more constant dosing to a patient.

Preferably the dissolution rates of the oral dosage forms of this invention are arranged, for example by routine adjustment of the erodable coating and dimensions of the opening(s), so that the rate of release is substantially similar in the different pH environments experienced by the dosage form on administration. Dissolution rates may be assessed by *in vitro* testing in solutions of the appropriate pHs. For example, when comparing dissolution in the stomach and intestine, tests may be carried out initially at pH 1.5 with a transfer to pH 6.8 after 2 hours or 4 hours, as an assumed time for residence in the stomach before emptying into the intestines of a notional patient in respectively fasted and fed conditions. Alternatively, tests may be carried out initially at pH 4.0, to simulate a fed stomach environment, with a transfer to pH 6.8 after 5 hours.

As mentioned above, Compound A or a pharmaceutically acceptable salt or solvate thereof when administered in an oral dosage form of this invention is indicated to be useful for the treatment and/or prophylaxis of diabetes mellitus, conditions associated with diabetes mellitus and certain complications thereof; metabolic syndrome, impaired glucose tolerance and impaired fasting glucose (hereinafter referred to as the Disorders of the Invention'). Suitably, Compound A or a pharmaceutically acceptable salt or solvate thereof when administered in an oral dosage form of this invention is indicated to be useful in the treatment and/or prophylaxis of diabetes mellitus, conditions associated with diabetes mellitus and certain complications thereof. Suitably, Compound A or a pharmaceutically acceptable salt or solvate thereof when administered in an oral dosage form of this invention is indicated to be useful in the treatment and/or prophylaxis of metabolic syndrome. Suitably, Compound A or a pharmaceutically acceptable salt or solvate thereof is indicated to be useful in the treatment and/or prophylaxis of impaired glucose tolerance. Suitably, Compound A or a pharmaceutically acceptable salt or solvate thereof when administered in an oral dosage form of this invention is indicated to be useful in the treatment and/or prophylaxis of impaired fasting glucose.

In a preferred embodiment the present invention provides a method for the treatment and/or prophylaxis of the Disorders of the Invention which method comprises administering an oral dosage form of this invention comprising Compound A or a pharmaceutically acceptable salt or solvate thereof and metformin or a pharmaceutically acceptable salt or solvate thereof, to a human or non-human mammal in need thereof.

In a further preferred embodiment the present invention provides an oral dosage form of the invention comprising Compound A or a pharmaceutically acceptable salt or solvate thereof and metformin or a pharmaceutically acceptable salt or solvate thereof for use in the treatment and/or prophylaxis of the Disorders of the Invention.

Suitable pharmaceutically acceptable forms of the other antidiabetic agent depend upon the particular agent used but included are known pharmaceutically acceptable forms of the particular agent chosen. Such derivatives are found or are referred to in standard reference texts such as the British and US Pharmacopoeias, Remington's Pharmaceutical Sciences (Mack Publishing Co.), The Extra Pharmacopoeia (London, The Pharmaceutical Press) (for example see the 31 st Edition page 341 and pages cited therein) and the above-mentioned publications. For example, a particular form of metformin is metformin hydrochloride.

As used herein the term "pharmaceutically acceptable" embraces compounds, compositions and ingredients for both human and veterinary use. For example the term "pharmaceutically acceptable salt" embraces a veterinarily acceptable salt. In particular, suitable pharmaceutically acceptable salted forms of Compound A include those described in European Patent Number 0 306 228 and International Patent Application, Publication Number WO 94/05659. A particularly preferred salt of Compound A is the maleate salt. A preferred pharmaceutically acceptable solvated form of Compound A is a hydrate.

As used herein, the term "Cₘₐₓ" shall mean the mean maximum plasma level concentration.

As used herein the term "AUC" shall mean the mean area under the plasma concentration versus time curve over the dosing interval at steady state.

No adverse toxicological effects are indicated in the above mentioned treatments.

In the following Examples, tablet cores were formed by conventional means by mixing together the active ingredients with excipients and compressing to form the tablet core. These Examples are intended to be by way of illustration rather than limitation of the present invention and the combination of Compound A and metformin is used simply as one example of a combination suitable for use with the present invention.

### Example 1

A core was formed from the following formulation:

| Immediate Release Laver | %w/w |
|---|---|
| Compound A (as maleate salt) | 0.5 |
| Compound B (as hydrochloride salt) | 85.2 |
| Lactose Monohydrate | 1.9 |
| Microcrystalline cellulose | 5.6 |
| Magnesium stearate | 0.5 |
| Hypromellose (HPMC) | 3.6 |
| Sodium starch glycolate | 0.2 |
| Povidone | 2.6 |

by compression to form a 19.0mm x 9.2mm, oval tablet of 1174 mg.

The tablet cores were coated with a HPMC-based sub-coat and a polymethacrylate resin soluble at pH 5.5 to a total weight of 1246.5 mg.

An opening of diameter 3.0 mm was drilled through the coating in each of the two primary surfaces of the coated cores to expose the surface of the core.

### Example 2

A core was formed from the following formulation:

| | %w/w |
|---|---|
| Immediate Release Layer | |
| Compound A (as maleate salt) | 0.5 |
| Compound B (as hydrochloride salt) | 85.2 |
| Lactose Monohydrate | 1.9 |
| Microcrystalline cellulose | 5.6 |
| Magnesium stearate | 0.5 |
| Hypromellose (HPMC) | 3.6 |
| Sodium starch glycolate | 0.2 |
| Povidone | 2.6 |

by compression to form a 19.0mm x 9.2mm, oval tablet of 1174 mg.

The tablet cores were coated with a HPMC-based sub-coat and a polymethacrylate resin soluble at pH 5.5 to a total weight of 1246.5 mg.

An opening of diameter 4.0 mm was drilled through the coating in each of the two primary surfaces of the coated cores to expose the surface of the core.

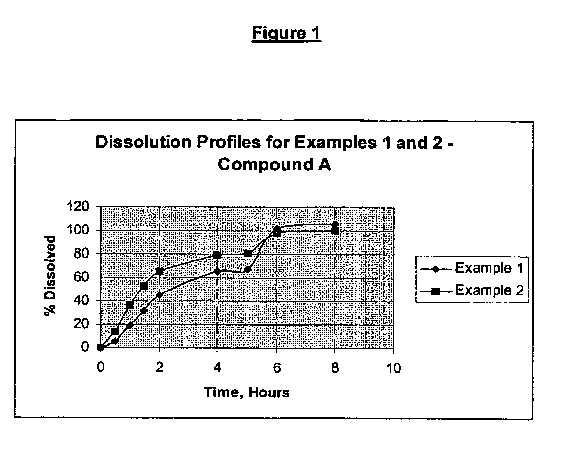

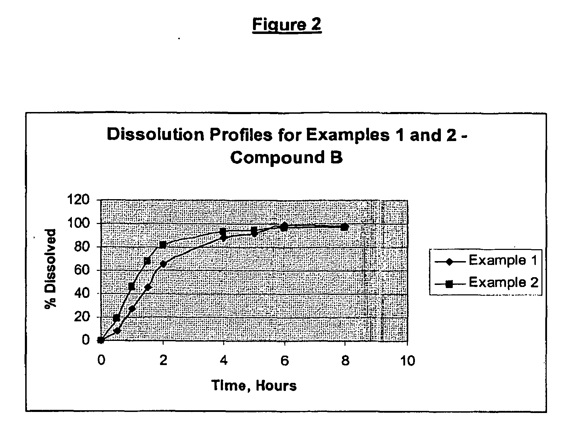

Dissolution profiles for the dosage forms of Examples 1 and 2, for Compound A and metformin ('Compound B') are shown in Figures 1 and 2 respectively in the accompanying drawings. Dissolution tests were performed initially at pH 4.0, with a transfer to pH 6.8 after 5 hours.

### A Study to Estimate the Pharmacokinetics of Six Extended Release Formulations of AVANDAMET™ (rosiglitazone maleate 4 mg/metformin HCl 1000 mg), Compared to the Commercial Formulation of AVANDAMET™ rosiglitazone maleate 2 mg/metformin HCl 500 mg, given twice daily), and Concomitant Dosing of Giucophaget XR (metformin HCl 2 x 500 mg) with AVANDIA™ (rosiglitazone maleate 4 mg)

### Primary Objectives

To compare the single dose pharmacokinetics of six extended release formulations of AVANDAMET™ (rosiglitazone maleate 4 mg/metformin HCl 1000 mg) to those of the commercial formulation of AVANDAMET™ (rosiglitazone maleate 2 mg/metformin HCl 500 mg, given twice daily). To compare the single dose pharmacokinetics of six extended release formulations of AVANDAMET™ (rosiglitazone maleate 4 mg/metformin HCl 1000 mg) to those of concomitantly dosed Glucophage XR (metformin HCl 2 × 500 mg) with AVANDIA™ (rosiglitazone maleate 4 mg).

### Secondary Objectives

To assess the safety and tolerability of single oral doses of each of the six extended release formulations of AVANDAMET™ (rosiglitazone maleate 4 mg/metformin HCl 1000 mg); currently marketed formulation of AVANDAMET™ (rosiglitazone maleate 2 mg/metformin 500 mg, given twice daily); and concomitant dosing of Glucophage XR (metformin hydrochloride 2 x 500 mg) with AVANDIA™ (rosiglitazone maleate 4 mg) commercial tablets. To compare the pharmacokinetics of the currently marketed formulation of AVANDAMET™ (rosiglitazone maleate 2 mg/metformin HCl 500 mg, given twice daily) to concomitantly dosed Glucophage XR (metformin HCl 2 x 500 mg) with AVANDIA™ (rosiglitazone maleate 4 mg).

### Study Design

This was a randomized, open-label, four-period, period-balanced crossover study with three parallel groups conducted in healthy volunteers. Each subject participated in four study sessions separated by a washout period of at least 7 days. In each study session, subjects were randomized to receive either a single oral dose of AVANDAMET™, AVANDIA™ plus Glucophage XR, or two of six extended release formulations of AVANDAMET™ in the evening under fed conditions.

### Number and nature of subjects

Fifty-one subjects were enrolled in the study and thirty-nine subjects completed the study. Subjects were healthy adult males and females between 18 and 65 years of age, inclusive, with body weight > 50 kg (110 lbs) and Body mass index (BMI) between 19 and 30 kg/m².

### Criteria for evaluation

Plasma specimens for rosiglitazone and metformin pharmacokinetic analysis were obtained prior to study medication administration in each session and over a 24-hour interval. Plasma concentration-time data were analyzed for rosiglitazone and metformin. The following pharmacokinetic parameters were determined, if data permitted: maximum observed plasma concentration (Cmax), time to Cmax (tmax), area under the plasma concentration-time curve to the last measurable concentration (AUC(0-t)), (AUC(0-36h)), and extrapolated to infinity (AUC(0-inf)), and half-life (t½).
Safety and tolerability were assessed by adverse events, clinical laboratory evaluations (hematology, clinical chemistry and urinalysis), vital signs (semi-recumbent blood pressure, heart rate), 12-lead ECG and concurrent medications. All subjects who received at least one dose of study medication were included in the evaluation of clinical safety and tolerability.

### Pharmacokinetic Results

### Geometric Mean (Range) Metformin Pharmacokinetic Parameters:

| **Regimen** | **Cmax, ng/mL** | **AUC(0-∞), ng.h/mL** | **AUC(0-t), ng.h/mL** | **Tmax¹, h** | **t½, h** |
|---|---|---|---|---|---|
| A: Commercial Avandamet, bid | 895 (565-1404) | 12330 (8868-18388) | 12010 (8353-18070) | 3.48 (1.50-6.00) | 4.24 (2.81-5.98) |
| B: Avandia+Glucophage XR | 1140 (412-2189) | 11904 (3252-20741) | 11614 (2993-20560) | 6.00 (2.50-10.00) | 5.63 (2.99-10.65) |
| C: Extended Release #1 (rosiglitazone 4mg / metformin 1000 mg) | 1074 (478-1845) | 9357 (4923-14160) | 8788 (4738-13992) | 10.00 (1.00-12.02) | 5.16 (4.33-7.00) |
| D: Extended Release #2 (rosiglitazone 4mg / metformin 1000 mg) | 1308 (940-1928) | 11178 (8573-15499) | 10787 (8191-15311) | 8.00 (5.00-10.00) | 5.27 (2.74-7.29) |
| E: Extended Release #3 (rosiglitazone 4mg / metformin 1000 mg) | 1382 (1016-1751) | 12995 (10116-16212) | 12727 (9991-15995) | 8.00 (2.80-10.00) | 4.87 (2.74-6.45) |
| F: Extended Release #4 (rosiglitazone 4mg / metformin 1000 mg) | 1372 (817-2447) | 12527 (7307-21615) | 12325 (7191-21339) | 8.00 (4.00-10.35) | 4.99 (3.41-6.72) |
| G: Extended Release #5 | 1472 | 13492 | 12926 | 6.00 | 5.69 |
| (rosiglitazone 4mg / metformin 1000 mg) | (911-1472) | (8529-18214) | (8201-17903) | (5.00-10.00) | (3.01-9.20) |
| H: Extended Release #6 (rosiglitazone 4mg / metformin 1000 mg) | 1363 (1010-2045) | 12440 (9206-19061) | 12235 (9061-18715) | 6.00 (3.00-8.00) | 5.57 (3.04-9.24) |

The plasma AUC observed for Compound A (rosiglitazone maleate) generated from Extended Release formulation #3 was equivalent to the rosiglitazone AUC from the reference regimens (i.e. A and B). Similarly, the plasma metformin AUC observed generated from Extended Release formulation #3 was equivalent to the metformin AUC from the reference regimens (i.e. A and B). Similar extended-release plasma concentration profiles were observed for both active agents. After administration of the extended release formulation #3, the observed inter-subject variability of rosiglitazone and metformin pharmacokinetic parameters was consistent with the reference treatment groups (regimens A and B).

### Conclusion

A once-a-day modified release tablet formulation comprising Compound A and metformin has been identified that on administration provides equivalent area under the plasma concentration versus time curve (after a single-dose) compared to the AUC observed after administration of the immediate release tablet formulation comprising Compound A and metformin (given bid x 2 doses). Furthermore, AUC equivalence was demonstrated between the modified release formulation (rosiglitazone and metformin) compared to a single dose of concomitantly administered rosiglitazone and glucophage XR.

## Claims

1. An oral dosage form comprising an erodable core which comprises 5-[4-[2-(N-methyl-N-(2 pyridyl)amino)ethoxy]benzyl]thiazolidine-2,4-dione or a Pharmaceutical acceptable salt or solvate thereof and metformin or a pharmaceutically acceptable salt or solvate thereof, the core having a coating with one or more openings leading to the core, **characterised in that** the coating is erodable under predetermined pH conditions.

2. An oral dosage form according to claim 1, comprising,
(i) an erodable core, which core comprises 5-[4-[2-(N-methyl-N-(2 pyridyl)amino)ethoxy]benzyl]thiazolidine-2,4-dione or a pharmaceutically acceptable salt or solvate thereof and metformin or a pharmaceutically acceptable salt or solvate thereof; and
(ii) an erodable coating around said core, which coating comprises one or more openings extending substantially completely through said coating but not substantially penetrating said core and communicating from the environment of use to said core;
wherein release of 5-[4-[2-(N-methyl-N-(2
pyridyl)amino)ethoxy]benzyl]thiazolidine-2,4-dione or a pharmaceutically acceptable salt or solvate thereof and metformin or a pharmaceutically acceptable salt or solvate thereof from the erodable core occurs substantially through the said opening(s) and through erosion of said erodable coating under pre-determined pH conditions.

3. An oral dosage form according to claim 1 or claim 2, wherein the erodable coating is an enteric coating.

4. An oral dosage form according to claim 3, wherein the enteric coating is non-permeable.

5. An oral dosage form according to claim 1, wherein the erodable core is formulated to provide immediate release of both 5-[4-[2-(N-methyl-N-(2 pyridyl)amino)etboxy]benzyl]thiazolidine-2,4-dione or a pharmaceutically acceptable salt or solvate thereof and metformin or a pharmaceutically acceptable salt or solvate thereof.

6. An oral dosage form according to any preceding claim, wherein said dosage form is a tablet form.

7. An oral dosage form according to any preceding claim, wherein the erodable coating erodes in the pH range from 4.5 to 8.

8. An oral dosage form according to any preceding claim, wherein the erodable coating erodes in the pH range from 5 to 7.

9. An oral dosage form according to claims 7 or 8, wherein the material used for the erodable coating is selected from polymethacrylate polymers, coprocessed polyvinylacetate phthalate, cellulose acetate trimellitate, celluloseacetate phthalate, hydroxypropylmethylcellulose phthalate polymers and their copolymers.

10. An oral dosage form according to claim 9, wherein said material is selected from cellulose acetate trimellitate, polyvinyl acetate phthalate, hydroxypropylmethylcellulose phthalate 55, Acryl-ezeTM, AquatericTM, cellulose acetate phthalate, Eudragit™ L30D, Eudragit™ L, Eudragit™ S and shellac.

11. An oral dosage form according to claim 10, wherein said material is Eudragit™ L30D.

12. An oral dosage form according to any preceding claim, which comprises 2 to 12mg of 5-[4-[2-(N-methyl-N-(2 pyridyl)amino)ethoxy]benzyl]thiazolidine-2,4-dione.

13. An oral dosage form according to claim 12, which comprises 4 or 8mg of 5-[4-[2-(N-methyl-N-(2 pyridyl)amino)ethoxy]benzyl]thiazolidine-2,4-dione.

14. An oral dosage form according to any preceding claim, which comprises 100 to 3000mg of metformin.

15. An oral dosage form according to claim 14, which comprises 500 or 1000mg of metformin.

16. An oral dosage form according to any preceding claim, wherein the 5-[4-[2-(N-methyl-N-(2 pyridyl)amino)ethoxy]benzyl]thiazolidine-2,4-dione is in the form of the maleate salt.

17. An oral dosage form according to any preceding claim, wherein the metformin is in the form of the hydrochloride salt.

18. An oral dosage form according to any preceding claim, wherein the typical sizes for the opening(s), when circular, to be formed in the coating are in the range 0.5 mm - 8 mm of diameter.

19. An oral dosage form according to any one of claims 1 to 17, wherein the areas of non-circular openings are in the range of from 0.19 to 50.3 mm².

20. An oral dosage form according to any preceding claim, wherein the erodable coating surrounding the core is provided with two openings extending substantially completely through said coating but not substantially penetrating said core and communicating from the environment of use to said core.

21. An oral dosage form according to claim 20, wherein the openings in the erodable coating are on opposing surfaces.

22. A process for the preparation of an oral dosage form according to claim 1, which process comprises:
(a) preparing an erodable tablet core comprising 5-[4-[2-(N-methyl-N-(2 pyridyl)amino)ethoxy]benzyl]thiazolidine-2,4-dione or a pharmaceutically acceptable salt or solvate thereof and met formin or a pharmaceutically acceptable salt or solvate thereof,
(b) coating the core with a material with pH-dependent erodability; and
(c) creating one or more openings in the coating.

## Patentansprüche

1. Eine orale Dosierungsform, umfassend einen erodierbaren Kern, der 5-[4-[2-(N-Methyl-N-(2-pyridyl)amino)ethoxy]benzyl]thiazolidin-2,4-dion oder ein pharmazeutisch verträgliches Salz oder Solvat davon und Metformin oder ein pharmazeutisch verträgliches Salz oder Solvat davon umfasst, wobei der Kern eine Beschichtung mit einer oder mehreren Öffnungen aufweist, die zu dem Kern führten, **dadurch gekennzeichnet, dass** die Beschichtung unter vorher festgelegten pH-Bedingungen erodierbar ist.

2. Eine orale Dosierungsform nach Anspruch 1, umfassend
(i) einen erodierbaren Kern, wobei der Kern 5-[4-[2-(N-Methyl-N-(2-pyridyl)amino)ethoxy]benzyl]thiazolidin-2,4-dion oder ein pharmazeutisch verträgliches Salz oder Solvat davon und Metformin oder ein pharmazeutisch verträgliches Salz oder Solvat davon umfasst; und
(ii) eine erodierbare Beschichtung um den Kern herum, wobei die Beschichtung eine oder mehrere Öffnungen umfasst, die sich im Wesentlichen vollständig durch die Beschichtung hindurch erstrecken, den Kern jedoch nicht wesentlich durchdringen und eine Verbindung zwischen der verwendeten Umgebung und dem Kern herstellen;
wobei die Freisetzung von 5-[4-[2-(N-Methyl-N-(2-pyridyl)ainino)ethoxy]benzyl]-thiazolidin-2,4-dion oder eines pharmazeutisch verträglichen Salzes oder Solvats davon und Metformin oder eines pharmazeutisch verträglichen Salzes oder Solvats davon aus dem erodierbaren Kern im Wesentlichen durch die Öffnung(en) und durch Erosion der erodierbaren Beschichtung unter vorher festgelegten pH-Bedingungen erfolgt.

3. Eine orale Dosierungsform nach Anspruch 1 oder Anspruch 2, wobei die erodierbare Beschichtung eine enterische Beschichtung ist.

4. Eine orale Dosienungsform nach Anspruch 3, wobei die enterische Beschichtung undurchlässig ist.

5. Eine orale Dosierungsform nach Anspruch 1, wobei der erodierbare Kern so formuliert ist, dass er eine unmittelbare Freisetzung von sowohl 5-[4-[2-(N-Methyl-N-(2-pyridyl)amino)ethoxy]benzyl]thiazolidin-2,4-dion oder eines pharmazeutisch verträglichen Salzes oder Solvats davon als auch von Metformin oder eines pharmazeutisch verträglichen Salzes oder Solvats davon bereitstellt.

6. Eine orale Dosierungsform nach einem vorhergehenden Anspruch, wobei die Dosierungsform eine Tablettenform ist.

7. Eine orale Dosierungsform nach einem vorhergehenden Anspruch, wobei die erodierbare Beschichtung im pH-Bereich von 4,5 bis 8 erodiert.

8. Eine orale Dosierungsform nach einem vorhergehenden Anspruch, wobei die erodierbare Beschichtung im pH-Bereich von 5 bis 7 erodiert.

9. Eine orale Dosierungsform nach den Ansprüchen 7 oder 8, wobei das für die erodierbare Beschichtung verwendete Material aus Polymethacrylat-Polymeren, gemeinsam verarbeiteten Polyvinylacetatphthalat-, Celluloseacetattrimellitat-, Celluloseacetatphthalat-, Hydroxypropylmethylcellulosephthalat-Polymeren und deren Copolymeren ausgewählt ist.

10. Eine orale Dosierungsform nach Anspruch 9, wobei das Material aus Celluloseacetattrimellitat, Polyvinylacetatphthalat, Hydroxypropylmethylcellulosephthalat 55, Acryl-eze TM, Aquateric TM, Celluloseacetatphthalat, Eudragit^{™} L30D, Eudragi^{™} L, Eudragit^{™} S und Schellack ausgewählt ist.

11. Eine orale Dosierungsform nach Anspruch 10, wobei das Material Eudragi^{™} L30D ist.

12. Eine orale Dosierungsform nach einem vorhergehenden Anspruch, welche 2 bis 12 mg 5-[4-[2-(N-Methyl-N-(2-pyridyl)amino)ethoxy]benzyl]thiazolidin-2,4-dion umfasst.

13. Eine orale Dosierungsform nach Anspruch 12, welche 4 oder 8 mg 5-[4-[2-(N-Methyl-N-(2-pyridyl)amino)ethoxy]benzyl]thiazolidin-2,4-dion umfasst.

14. Eine orale Dosierungsform nach einem vorhergehenden Anspruch, welche 100 bis 3000 mg Metfonmin umfasst.

15. Eine orale Dosierungsform nach Anspruch 14, welche 500 oder 1000 mg Metformin umfasst.

16. Eine orale Dosierungsform nach einem vorhergehenden Anspruch, wobei das 5-[4-[2-(N-Methyl-N-(2-pyridyl)amino)ethoxy]benzyl]thiazolidin-2,4-dion in Form des Maleatsalzes vorliegt.

17. Eine orale Dosierungsform nach einem vorhergehenden Anspruch, wobei das Metformin in Form des Hydrochloridsalzes vorliegt.

18. Eine orale Dosierungsform nach einem vorhergehenden Anspruch, wobei die typischen Größen für die in der Beschichtung zu bildende(n) Öffnung(en), wenn sie kreisförmig ist/sind, im Bereich von 0,5 mm - 8 mm Durchmesser liegen.

19. Eine orale Dosierungsform nach einem der Ansprüche 1 bis 17, wobei die Flächen nichtkreisförmiger Öffnungen im Bereich von 0,19 bis 50,3 mm² liegen.

20. Eine orale Dosierungsform nach einem vorhergehenden Anspruch, wobei die erodierbare Beschichtung, die den Kern umgibt, mit zwei Öffnungen versehen ist, die sich im Wesentlichen vollständig durch die Beschichtung hindurch erstrecken, den Kern jedoch nicht wesentlich durchdringen und eine Verbindung zwischen der verwendeten Umgebung und dem Kern herstellen.

21. Eine orale Dosierungsform nach Anspruch 20, wobei sich die Öffnungen in der erodierbaren Beschichtung auf gegenüberliegenden Oberflächen befinden.

22. Ein Verfahren zur Herstellung einer oralen Dosierungsform nach Anspruch 1, wobei das Verfahren umfasst:
(a) Herstellen eines erodierbaren Tablettenkerns, umfassend 5-[4-[2-(N-Methyl-N-(2-pyridyl)amino)ethoxy]benzyl]thiazolidin-2,4-dion oder ein pharmazeutisch verträgliches Salz oder Solvat davon und Metformin oder ein pharmazeutisch verträgliches Salz oder Solvat davon,
(b) Beschichten des Kerns mit einem Material mit pH-abhängiger Erodierbarkeit; und
(c) Bereitstellen einer oder mehrerer Öffnungen in der Beschichtung.

## Revendications

1. Forme posologique orale comprenant un noyau érodable qui comprend de la 5-[4-[2-(N-méthyl-N-(2-pyridyl)amino)-éthoxy]benzyl]thiazolidine-2,4-dione ou un de ses sels ou produits de solvatation pharmaceutiquement acceptables et de la metformine ou un de ses sels ou produits de solvatation pharmaceutiquement acceptables, le noyau comportant un enrobage avec un ou plusieurs orifices conduisant au noyau, **caractérisé en ce que** l'enrobage est érodable dans des conditions de pH prédéterminées.

2. Forme posologique orale suivant la revendication 1, comprenant
(i) un noyau érodable, noyau qui comprend de la 5-[4-(2-(N-méthyl-N-(2-pyridyl)amino)éthoxy]benzyl]thiazolidine-2,4-dione ou un de ses sels ou produits de solvatation pharmaceutiquement acceptables et de la metformine ou un de ses sels ou produits de solvatation pharmaceutiquement acceptables ; et
(ii) un enrobage érodable autour dudit noyau, enrobage qui comprend un ou plusieurs orifices s'étendant substantiellement totalement à travers ledit enrobage mais ne pénétrant pas substantiellement dans ledit noyau et assurant la communication de l'environnement d'utilisation audit noyau ;
dans laquelle la libération de la 5-[4-[2-(N-méthyl-N-(2-pyridyl)amino)éthoxy]benzyl]thiazolidine-2,4-dione ou d'un de ses sels ou produits de solvatation pharmaceutiquement acceptables et de la metformine ou d'un de ses sels ou produits de solvatation pharmaceutiquement acceptables par le noyau érodable se produit substantiellement à travers ledit ou lesdits orifices et par érosion dudit enrobage érodable dans des conditions de pH prédéterminées.

3. Forme posologique orale suivant la revendication 1 ou la revendication 2, dans laquelle l'enrobage érodable est un enrobage entérique.

4. Forme posologique orale suivant la revendication 1,
dans laquelle l'enrobage entérique est non perméable.

5. Forme posologique orale suivant la revendication 1,
dans laquelle le noyau érodable est formulé pour provoquer la libération immédiate à la fois de la 5-[4-[2-(N-méthyl-N-(2-pyridyl)amino)éthoxy]benzyl]thiazolidine-2,4-dione ou d'un de ses sels ou produits de solvatation pharmaceutiquement acceptables et de la metformine ou d'un de ses sels ou produits de solvatation pharmaceutiquement acceptables.

6. Forme posologique orale suivant l'une quelconque des revendications précédentes, ladite forme posologique étant sous forme de comprimé.

7. Forme posologique orale suivant l'une quelconque des revendications précédentes, dans laquelle l'enrobage érodable s'érode à un pH compris dans la plage de 4,5 à 8.

8. Forme posologique orale suivant l'une quelconque des revendications précédentes, dans laquelle l'enrobage érodable s'érode à un pH compris dans la plage de 5 à 7.

9. Forme posologique orale suivant la revendication 7 ou 8, dans laquelle la matière utilisée pour l'enrobage érodable est choisie entre des polymères du type polyméthacrylate, le polyacétophtalate de vinyle co-traité, l'acétotrimellitate de cellulose, l'acétophtalate de cellulose, des polymères de phtalate d'hydroxypropylméthylcellulose et leurs copolymères.

10. Forme posologique orale suivant la revendication 9,
dans laquelle ladite matière est choisie entre l'acétotrimellitate de cellulose, l'acétophtalate de polyvinyle, le phtalate d'hydroxypropylméthylcellulose 55, l'Acryl-eze TM, l'Aquateric TM, l'acétophtalate de cellulose, l'Eudragit™ L30D, l'Eudragit™ L, l'Eudragit™ S et la gomme laque.

11. Forme posologique orale suivant la revendication 10,
dans laquelle ladite matière est l'Eudragit™ L30D.

12. Forme posologique orale suivant l'une quelconque des revendications précédentes, qui comprend 2 à 12 mg de 5-[4-[2-(N-méthyl-N-(2-pyridyl)amino)éthoxy]benzyl]thiazolidine-2,4-dione.

13. Forme posologique orale suivant la revendication 12, qui comprend 4 ou 8 mg de 5-[4-[2-(N-méthyl-N-(2-pyridyl)-amino)éthoxy]benzyl]thiazolidine-2,4-dione.

14. Forme posologique orale suivant l'une quelconque des revendications précédentes, qui comprend 100 à 300 mg de metformine.

15. Forme posologique orale suivant la revendication 14, qui comprend 500 ou 1000 mg de metformine.

16. Forme posologique orale suivant l'une quelconque des revendications précédentes, dans laquelle la 5-[4-(2-(N-méthyl-N-(2-pyridyl)amino)éthoxy]benzyl]thiazolidine-2,4-dione est sous forme du sel consistant en maléate.

17. Forme posologique orale suivant l'une quelconque des revendications précédentes, dans laquelle la metformine est sous forme du sel consistant en chlorhydrate.

18. Forme posologique orale suivant l'une quelconque des revendications précédentes, dans laquelle les dimensions classiques du ou des orifices, lorsqu'ils sont circulaires, à former dans l'enrobage sont comprises dans l'intervalle de 0,5 mm à 8 mm de diamètre.

19. Forme posologique orale suivant l'une quelconque des revendications 1 à 17, dans laquelle les surfaces des orifices non circulaires sont comprises dans l'intervalle de 0,19 à 50, 3 mm².

20. Forme posologique orale suivant l'une quelconque des revendications précédentes, dans laquelle l'enrobage érodable entourant le noyau est muni de deux orifices s'étendant substantiellement totalement à travers ledit enrobage mais ne pénétrant pas substantiellement dans ledit noyau et assurant la communication de l'environnement d'utilisation audit noyau.

21. Forme posologique orale suivant la revendication 20,
dans laquelle les orifices dans l'enrobage érodable sont sur des surfaces opposées.

22. Procédé pour la préparation d'une forme posologique orale suivant la revendication 1, procédé qui comprend :
(a) la préparation d'un noyau de comprimé érodable comprenant de la 5-[4-[2-(N-méthyl-N-(2-pyridyl)amino)éthoxy]-benzyl]thiazolidine-2,4-dione ou un de ses sels ou produits de solvatation pharmaceutiquement acceptables et de la metformine ou un de ses sels ou produits de solvatation pharmaceutiquement acceptables ;
(b) l'enrobage du noyau avec une matière présentant une capacité d'érosion dépendant du pH ; et
(c) la création d'un ou plusieurs orifices dans l'enrobage.
